(19) Europäisches Patentamt / European Patent Office / Office européen des brevets

(11) **EP 3 684 249 B1**

(12) **EUROPEAN PATENT SPECIFICATION**

(45) Date of publication and mention of the grant of the patent:
**15.04.2026 Bulletin 2026/16**

(21) Application number: **18769305.6**

(22) Date of filing: **05.09.2018**

(51) International Patent Classification (IPC):
*A61B 5/02* (2006.01)     *A61B 5/029* (2006.01)
*A61B 5/053* (2021.01)

(52) Cooperative Patent Classification (CPC):
**A61B 5/0535; A61B 5/02028; A61B 5/029**

(86) International application number:
**PCT/EP2018/073799**

(87) International publication number:
**WO 2019/057488 (28.03.2019 Gazette 2019/13)**

(54) **SYSTEM AND METHOD FOR ESTIMATING THE STROKE VOLUME AND/OR THE CARDIAC OUTPUT OF A PATIENT**

SYSTEM UND VERFAHREN ZUR SCHÄTZUNG DES SCHLAGVOLUMENS UND/ODER DER HERZLEISTUNG EINES PATIENTEN

SYSTÈME ET PROCÉDÉ PERMETTANT D'ESTIMER LE VOLUME SYSTOLIQUE ET/OU LE DÉBIT CARDIAQUE D'UN PATIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.09.2017 EP 17382625**

(43) Date of publication of application:
**29.07.2020 Bulletin 2020/31**

(73) Proprietor: **Quantium Medical S.L.U.**
**08302 Mataró (Barcelona) (ES)**

(72) Inventors:
• **MUNOZ, Jesus, Escriva**
 **08302 Mataro Catalunya (ES)**
• **JENSEN, Erik, Weber**
 **08395 Sant Pol de Mar (ES)**

(74) Representative: **Fresenius Kabi Deutschland GmbH**
**Patent Department - MedTech**
**Else-Kröner-Straße 1**
**61352 Bad Homburg (DE)**

(56) References cited:
**WO-A1-2015/086020**     **US-A- 5 423 326**
**US-A- 5 443 073**

• **WANG X ET AL: "AN ADVANCED SIGNAL PROCESSING TECHNIQUE FOR IMPEDANCE CARDIOGRAPHY", IEEE TRANSACTIONS ON BIOMEDICAL ENGINEERING, IEEE SERVICE CENTER, PISCATAWAY, NJ, USA, vol. 42, no. 2, 1 February 1995 (1995-02-01), pages 224 - 230, XP000556799, ISSN: 0018-9294, DOI: 10.1109/ 10.341836**
• **CARRASCO-SOSA SALVADOR ET AL: "Variability of the maximal amplitudes of impedance cardiogram and of its first derivative during supine, standing, paced breathing, and exercise maneuvers", COMPUTING IN CARDIOLOGY 2013, N/A, 7 September 2014 (2014-09-07), pages 1065 - 1068, XP032737147, ISSN: 2325-8861, ISBN: 978-1-4799-0884-4, [retrieved on 20150217]**

**Description**

The invention relates to a system for estimating the stroke volume and/or the cardiac output of a patient according to the preamble of claim 1 and to a method for estimating the stroke volume and/or the cardiac output of a patient.

A system of this kind comprises a processor device constituted to receive a bio-impedance measurement signal relating to a bio-impedance measurement on the thorax of a patient, to process the bio-impedance measurement signal to extract a group of characteristic features from the bio-impedance measurement signal and/or its derivative, and to determine, using the group of expected features, an output indicative of the stroke volume and/or the cardiac output using at least one non-linear model.

Generally, a patient's haemodynamic status may change rapidly, such that a frequent or even continuous monitoring of cardiac output may provide useful information allowing for a rapid reaction and potentially an adjustment of therapy if needed.

A common method for monitoring cardiac output (CO) is the thermodilution technique executed using a pulmonary artery catheter (PAC). The PAC is also termed a Swan-Ganz catheter, named after the inventors of the technique. The PAC is introduced into the vena cava, and then fed through the heart to position the tip of the catheter in the pulmonary artery. The long history of its use has led to much experience with this technology and its clinical application.

As an alternative method, the bio-impedance method was introduced as a simple, lowcost method that provides information about the cardiovascular system and/or (de)-hydration status of the body in a non-invasive way. To improve the related thoracic impedance method, different thoracic impedance measurement systems have been proposed to determine the stroke volume (SV) or cardiac output on a beat-to-beat time base. A large number of validation studies have been reported, with different results compared to a reference method. The accuracy of bio-impedance measurements may be increased by placing electrodes directly in the left ventricle, rather than on the chest (thorax) of a patient. Alternatively, accuracy can be improved by applying advanced signal processing or combining several parameters into a final estimate of the cardiac output.

The measuring of bio-impedance on body parts is for example described in US 3,340,867, which discloses a so-called impedance plethysmography in particular useful for determining cardiac output.

US 3,835,840 describes an impedance plethysmography apparatus and method for using the electrical impedance as a correlate to blood flow in the aorta or other arteries.

WO 2015/086020 A1 describes an apparatus for determining stroke volume, cardiac output and systemic inflammation by a fuzzy logic combination of characteristic features extracted from a voltage measured over the thorax, electrocardiogram and electroencephalogram.

It is an object of the instant invention to provide a system and a method for estimating the stroke volume and/or the cardiac output of a patient, which allow a reliable and precise estimation of the stroke volume and/or the cardiac output of a patient using signal analysis of a bio-impedance measurement signal.

This object is achieved by means of a system comprising the features of claim 1.

Accordingly, the processor device is constituted to process the bio-impedance measurement signal to compute at least one time-frequency distribution based on the bio-impedance measurement signal and/or the derivative of the bio-impedance measurement signal and to determine at least one characteristic feature of said group of characteristic features based on the at least one time-frequency distribution.

The computation of the at least one time-frequency distribution is generally based on the bio-impedance measurement signal, which may include any processing of the bio-impedance measurement signal, in particular the computation of the derivative of the bio-impedance measurement signal.

By determining one or multiple time-frequency distributions relating to the bio-impedance measurement signal, characteristic features may be extracted and determined from the bio-impedance measurement signal which may allow to determine, using a subsequent processing, a precise estimate of the stroke volume and/or the cardiac output of a patient.

By means of the computation of time-frequency distributions, in particular several parameters related to the cyclic behavior of the signal and/or related to physiological variables in the patient (for example relating to the stroke volume and cardiac output) may be extracted from the bio-impedance measurement signal (which in one embodiment is a voltage curve) or its derivative.

The time-frequency distribution of a signal generally is defined as

$$\rho(t,f) = \iint G(t-u,\tau)z\left(u+\frac{\tau}{2}\right)\bar{z}\left(u-\frac{\tau}{2}\right)dud\tau$$

in which $\rho$ represents the time-frequency distribution, $G(t, \tau)$ represents a time-lag kernel, $z$ represents the analytic associate of the bio-impedance measurement signal to be analysed, and $\bar{z}$ represents the complex conjugate of $z$. The analytic associate of the bio-impedance measurement signal is calculated as $z(t) = x(t) + j\mathcal{H}\{x(t)\}$, where $\mathcal{H}\{x(t)\}$ is the Hilbert transform of said bio-impedance measurement signal. The time-lag kernel $G(t, \tau)$ determines the characteristics of the time-frequency distributions and how the signal energy is distributed in the time-frequency plane. Different time-lag kernels which may be applicable in the instant case have been presented in the literature (see for example B. Boashash, "Time-Frequency Signal Analysis and Processing: a Comprehensive Reference", Elsevier 2003). The Wigner-Ville Distribution (choosing G = 1) is the most basic kernel. Other well-known distributions which can be applied in the instant case are the so-called Modified Beta Distribution, the Zhao-Atlas-Marks Distributions, the Born-Jordan Distribution, wherein also other distributions as defined in the literature are possible and applicable here.

For determining one or multiple characteristic features relating to the measurement signal obtained, the processor device may be constituted to determine different time-frequency distribution features from one or multiple time-frequency distributions. The time-frequency distribution features may in particular include:

- the time-frequency complexity (TFC): representing the magnitude and number of the non-zero singular values of the time-frequency distribution in a Singular Value Decomposition (SVD; the SVD magnitudes have a strong relationship with the information content in the time-frequency distribution);
- the time-frequency Rényi entropy (TFRE) and normalized time-frequency Rényi entropy (NTFRE): a useful classification measure for non-stationary signals because of its sensitivity of signal components, their duration and bandwidth;
- the energy concentration measure (ECOME): determining the concentration of the dominant component at each location in the TF domain; and
- the (momentary) energy in one or multiple different frequency bands.

Within the instant concept, the time-frequency features may directly be used as characteristic features for determining values indicative of the stroke volume and/or the cardiac output of a patient. It however is also conceivable to process the time-frequency distribution features further in order to derive one or multiple characteristic features from the time-frequency distribution features, for example by combining two or more time-frequency distribution features to obtain a suitable characteristic features to be used for determining the stroke volume and/or the cardiac output. The combination of the time-frequency distribution features may for example take place by using a suitable model (such as a generalized linear model, for example a quadratic model), which outputs a combined feature representing a characteristic feature for determining output values indicative of the stroke volume and/or the cardiac output.

The system furthermore includes two excitation electrodes to be placed on the thorax of a patient for applying an excitation signal, and two sensing electrodes to be placed on the thorax of the patient for sensing the bio-impedance measurement signal caused by the excitation signal. The two excitation electrodes are controlled to inject an electrical current having one predetermined frequencies and having a constant amplitude. Hence, via the two excitation electrodes a current is injected, which flows through a region of the patient and causes a voltage signal, which can be picked up by two sensing electrodes as the measurement signal. The voltage signal, also denoted as the voltage plethysmographic curve, voltage plethysmogram or voltage curve, is linked to the injected current via the bio-impedance, which in particular is influenced by blood flowing through the arteries of the patient's body on which the at least two excitation electrodes and the at least two sensing electrodes are placed.

The excitation signal excited by the at least two electrodes is an electrical current which is injected at a predetermined frequency and at constant amplitude. Two excitation electrodes, are placed on the thorax of the patient to let a current flow from one excitation electrode to the other. By means of the two sensing electrodes, then, a voltage signal can be detected which is linked to the injected excitation current by the bio-impedance of the patient.

The excitation current has a constant amplitude of 50 to 1000 $\mu$A and has a high frequency, for example 50 kHz.

Two excitation electrodes are placed on the thorax of the patient, one excitation electrode at an upper position on the thorax and another excitation electrode at a lower position on the thorax of the patient. The current hence flows in between the two excitation electrodes along the path of least resistance (impedance), i.e., along the blood-filled arteries within the thorax of the patient. In addition, two sensing electrodes are used, each sensing electrode being placed in the neighbourhood of one excitation electrode on the thorax of the patient.

The processor device furthermore is constituted to receive an electrocardiogram signal and to process the electrocardiogram signal to extract at least one characteristic feature to be used for determining output values indicative of the stroke volume and/or the cardiac output. Hence, characteristic features derived from a bio-impedance measurement signal are combined with characteristic features derived from an electrocardiogram signal, which may furthermore help to determine an accurate estimate of the stroke volume and/or the cardiac output.

Herein, the electrocardiogram signal is picked up by the same sensing electrodes which are also used to record the bio-impedance measurement signal. Hence, no additional electrodes are needed for measuring the electrocardiogram signal, but a common set up of sensing electrodes is used both for measuring the electrocardiogram signal and the bio-impedance measurement signal, wherein also a common processing of the signals may be used within the processor device.

In one embodiment, both the bio-impedance measurement signal and the electrocardiogram signal, sensed by a common set of sensing electrodes, are processed in the processor device in a processing path comprising an amplification device for amplifying the measurement signal and an analog-to-digital converter for digitizing the measurement signal. The amplification device in particular may be a low-noise amplifier (LNA) for amplifying the combined measurement signal (including the bio-impedance measurement signal and the electrocardiogram signal picked up by the sensing electrodes). By means of the analog-to-digital converter the (amplified) measurement signal is digitized for the further processing such that the further processing takes place on a digitized version of the measurement signal.

According to another aspect, the processor device may be constituted to extract one or multiple features of the group of a maximum value of the derivative of the bio-impedance measurement signal, a minimum value of the derivative of the bio-impedance measurement signal, a maximum amplitude of the bio-impedance measurement signal, a minimum amplitude of the bio-impedance measurement signal, a value of the left ventricular ejection time derived from the derivative of the bio-impedance measurement signal, an area obtained by integrating the derivative of the voltage curve over the left ventricular ejection time, and a value indicative of a time difference of an C peak in the derivative of the bio-impedance measurement signal and an R peak of an electrocardiogram signal. In particular, features may be extracted from the bio-impedance measurement signal as such, from a comparison of the bio-impedance measurement signal and the electrocardiogram signal, or from the electrocardiogram signal as such. The extracted features may then be used as characteristic features to determine output values indicative of the stroke volume and/or the cardiac output of the patient.

In one embodiment, the processor device may be constituted to feed the group of extracted features into a first non-linear model. Making use of the first non-linear model, the extracted characteristic features may for example be combined to output a value indicative of the stroke volume and, in addition, potentially an output value indicative of the probability of hypotension, the so-called hypotension index.

The non-linear model may for example be a fuzzy logic model, which may be trained, in an initial phase, according to training data for which the stroke volume is known. Within the training phase the parameters of the model are defined in a way that the model, being fed with input values correlating to the stroke volume, provides for an (accurate) estimate of the actual value of the stroke volume and/or probability of hypotension.

The further processing may for example make use of a correlate of the cardiac output, which may be determined by multiplying the value indicative of the stroke volume (obtained as output from the first non-linear model) with a value indicative of the heart rate of the patient. The value indicative of the heart rate of the patient may for example be derived from a periodicity detected in the bio-impedance measurement signal, or from the electrocardiogram signal, in particular according to C and/or R peaks detected in the bio-impedance measurement signal and/or the electrocardiogram signal.

The output value of the first non-linear model being indicative of the stroke volume and potentially in addition the correlate of the cardiac output may be fed to a second non-linear model, which also may be a fuzzy logic model or a quadratic equation model, the second non-linear model being constituted to process and combine the value indicative of the stroke volume with other input values to output a final output value indicative of the cardiac output and potentially also a final output value for the stroke volume. Further inputs to the second non-linear model may for example relate to information relating to the patient, for example the patient's weight, height, gender or age. Furthermore, a value indicative of the length of the trunk of the patient, correlated to the distance between an upper pair of electrodes and a lower pair of electrodes placed on the thorax of the patient, may be fed into the second non-linear model.

The second non-linear model may take as input furthermore a value indicative of the RR and/or CC interval (which is the inverse of the heart rate). Herein, the processor device may be constituted to derive a first value indicative of the CC interval from the bio-impedance measurement signal and a second value indicative of the RR interval from the electrocardiogram signal. The processor device may then compare the two values to each other in order to assess correct performance of the system, wherein a correction mechanism may be activated if it is found that the two values diverge from each other by more than a predefined threshold.

The object is also achieved by a method for estimating the stroke volume and/or the cardiac output of a patient, the method comprising:

- receiving a bio-impedance measurement signal relating to a bio-impedance measurement on the thorax of a patient,
- processing the bio-impedance measurement signal to extract a group of characteristic features from the bio-impedance measurement signal and/or its derivative, and
- determining, using the group of extracted characteristic features, an output indicative of the stroke volume and/or the cardiac output using at least one non-linear model.

The method further comprises the step of positioning two excitation electrodes (100E) on the thorax (20) of a patient (2) for applying an excitation signal, wherein the excitation electrodes (100E) are controlled to inject an electrical current having a constant amplitude of 50 to 1000 μA and a high frequency, for example 50 kHz, and at least two sensing electrodes (100S) to be placed on the thorax (20) of the patient (2) for sensing the bio-impedance measurement signal (VC) caused by the excitation signal, one excitation electrode (100E) is placed at an upper position, close to the neck of the patient, and another excitation electrode (100E) is placed at a lower position on the thorax, with each sensing electrode (100S) being arranged in the vicinity of the associated excitation electrode (100E), and the step of receiving by the processor device (12) electrocardiogram signal (ECG) and processing the electrocardiogram signal (ECG) to extract at least one characteristic feature, wherein the electrocardiogram signal (ECG) and the bio-impedance measurement signal (VC) are sensed using the same two common sensing electrodes (100S).

Herein, the processing of the bio-impedance measurement signal includes: processing the bio-impedance measurement signal to compute at least one time-frequency distribution based on the bio-impedance measurement signal and/or the derivative of the bio-impedance measurement signal, and determining at least one characteristic feature distribution based on the bio-impedance measurement signal and/or the derivative of the bio-impedance measurement signal, and determining at least one characteristic feature of said group of characteristic features based on the at least one time-frequency distribution.

The advantages and advantageous embodiments described above for the system equally apply also to the method.

The idea underlying the invention shall subsequently be described in more detail by referring to the embodiments shown in the figures. Herein:

Fig. 1          shows a schematic chart of a system for estimating the stroke volume and/or the cardiac output of a patient;

Fig. 2A         shows a diagram showing a bio-impedance measurement signal in the shape of a voltage signal;

Fig. 2B         shows a diagram of the derivative of the bio-impedance measurement signal in the shape of the voltage signal;

Fig. 2C         shows an electrocardiogram signal;

Fig. 3          shows a schematic view of a feature extraction unit, constituted to compute a time-frequency distribution to extract time-frequency distribution features from the bio-impedance measurement signal;

Fig. 4          shows a detailed view of non-linear models used to process characteristic features extracted from the bio-impedance measurement signal and the electrocardiogram signal to determine output values indicative of the stroke volume and the cardiac output; and

Fig. 5A, 5B     show a mathematical formulation of an ANFIS non-linear model.

The term "electrocardiography (ECG)" in this text refers to a transthoracic (across the thorax or chest) interpretation of the electrical activity of the heart over a period of time, as detected by electrodes attached to the surface of the skin and recorded by a device external to the body. The recording produced by this non-invasive procedure is termed an electrocardiogram. An ECG picks up electrical impulses generated by the depolarization of cardiac tissue and translates these into a waveform. The waveform is then used to measure the rate and regularity of heartbeats, as well as the size and position of the chambers, the presence of any damage to the heart, and the effects of drugs or devices used to regulate the heart, such as a pacemaker.

The term "fast Fourier transform (FFT)" in this text refers to an algorithm to compute the discrete Fourier transform (DFT) and its inverse. A Fourier transform converts time (or space) to frequency and vice versa; an FFT rapidly computes such transformations. As a result, fast Fourier transforms are widely used for many applications in engineering, science, and mathematics.

The term "RR intervals" in this text refers to the time between successive R-peaks in the ECG. From the RR intervals the following parameters may be extracted, as shown in Table 1 below. Herein, NNi refers to the i-th RR interval.

**Table 1. Time and frequency domain variables.**

| Variable | Description |
|---|---|
| HR (bpm) | Heart rate, reciprocate of the mean of all RR intervals |
| RMSSD (ms) | Root mean square differences between successive RR intervals |

(continued)

| Variable | Description |
|---|---|

$$RMSSD = \sqrt{\frac{1}{NN}\sum_i (NN_i - NN_{i-1})^2}$$

| Variable | Description |
|---|---|
| SDSD (ms) | Standard deviation of differences between successive RR intervals |
| pNN50 (%) | Number of interval differences of successive RR intervals greater than 50 ms divided by the total number of RR intervals., i.e: if ($NN_i$ - $NN_{i-1}$) > 50ms, *count ++;* count/n * 100; |
| HF ($ms^2$) | Power in high frequency range (0.15 - 0.4 Hz) |
| HFn (n.u) | HF power in normalized units, HFn = HF/(LF + HF)*100 |
| LF | Power in low frequency range (0 - 0.14 Hz) |

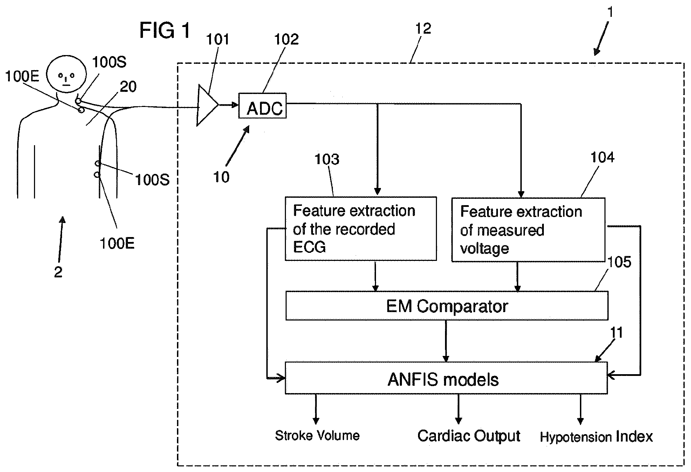

Fig. 1, in a schematic drawing, shows a system 1 for determining estimate values of the stroke volume (SV) and/or the cardiac output (CO) of a patient 2.

Within the system 1, different types of signals are combined in a processor device 12 making use of different non-linear models 11 in order to derive, from the input values, output values indicative of the stroke volume, the cardiac output and potentially also a hypotension index relating to the probability of hypotension.

The system 1 may be constituted as a computing device, for example a work station. The different units of the processor device 12 herein may be implemented by one or multiple hardware units or by software.

Within the system 1, in particular information derived from a measurement signal obtained from bio-impedance measurements and information obtained from an electrocardiogram (ECG) signal are combined. For this, the processor device 12 is constituted to process, in a processing path 10, electrocardiogram signals and bio-impedance measurement signals and to combine such different signals, in particular characteristic features extracted from such signals, in a model unit 11 to output values indicative of the stroke volume, the cardiac output and the hypotension index.

The electrocardiogram signals and the bio-impedance measurement signals are picked up and recorded, in the embodiment illustrated in Fig. 1, by common sensing electrodes 100S. By means of the sensing electrodes 100S, electrocardiogram signals relating to be spontaneous heart activity of the patient 2 and bio-impedance measurement signals are picked up and amplified in an amplification unit 101 (in particular a low-noise amplifier) of the processing path 10, after which the signals are fed into an analog-to-digital converter 102 for digitizing the signals.

For the bio-impedance measurements, in addition to the sensing electrodes 100S, excitation electrodes 100E are placed on the thorax 20 of the patient 2, as it is shown by way of example in Fig. 1. One excitation electrode 100E herein is placed at an upper position (for example close to the neck of the patient 2), and another excitation electrode 100E is placed at a lower position on the thorax, and an excitation signal in the shape of a constant current at an elevated frequency is injected in between the excitation electrodes 100E to flow through the patient's thorax 20. The excitation current may for example have a (constant) amplitude in the range between 50 and 1000 μA, for example 400 μA.

By the excitation current, which seeks its path through the patient's thorax 20 in particular along the blood-filled arteries within the patient's thorax 20, a voltage signal is caused which is linked to the injected current via the bio-impedance. This voltage signal is picked up by the two sensing electrodes 100S, each sensing electrode 100S being arranged in the vicinity of an associated excitation electrode 100E, as it is shown in Fig. 1.

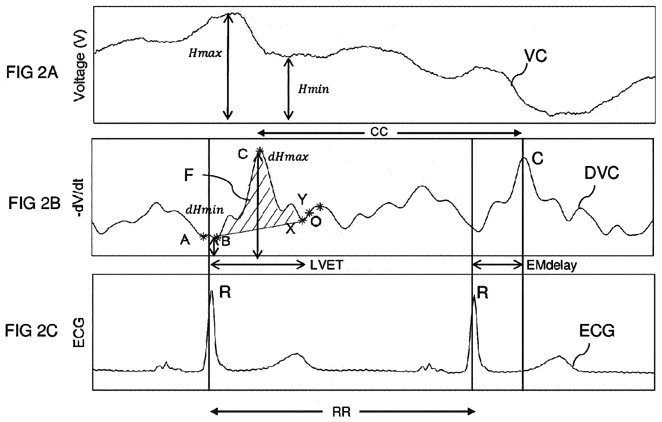

A bio-impedance measurement signal in the shape of a voltage curve VC as a function of time is shown in an example in Fig. 2A. Fig. 2B shows the derivative DVC of the voltage curve VC of Fig. 2A. And Fig. 2C shows an electrocardiogram signal E as picked up via the common sensing electrodes 100S in correlation to the voltage curve VC.

The processing within the processor device 12, as illustrated in Fig. 1, takes place on digitized versions of the measurement signal output from the analog-to-digital converter 102. For the processing, the bio-impedance measurement signal and the electrocardiogram signal each are each fed to a specific feature extraction unit 103, 104 to extract characteristic features from the electrocardiogram signal and the bio-impedance measurement signal.

Since electrocardiogram signals are present in base band and bio-impedance signals are modulated for example at 50 kHz, it is possible to pick up the electrocardiogram signals and the bio-impedance measurement signals with only two pairs of electrodes and to separate the signals for a further processing.

Within the feature extraction units 103, 104 characteristic features are extracted from the recorded electrocardiogram signal E (see Fig. 2C) and the voltage curve VC of the bio-impedance measurement signal (see Fig. 2A) as well as the derivative DVC of the voltage curve VC (see Fig. 2B).

Among the characteristic features extracted from the voltage curve VC of the bio-impedance measurement signal and the derivative DVC of the voltage curve VC may be in particular a maximum amplitude Hmax of the voltage signal VC and a minimum amplitude Hmin of the voltage signal VC (within the opening time of the aortic valve LVET) as determined from the voltage curve VC (see Fig. 2A), a maximum value of the derivative dHmax of the voltage curve VC and a minimum value of the derivative dHmin of the voltage curve VC (see Fig. 2B), the time period LVET of the opening time of the aortic valve (see Fig. 2B), and the area F under the voltage curve VC within the time period LVET of the opening time of the aortic valve.

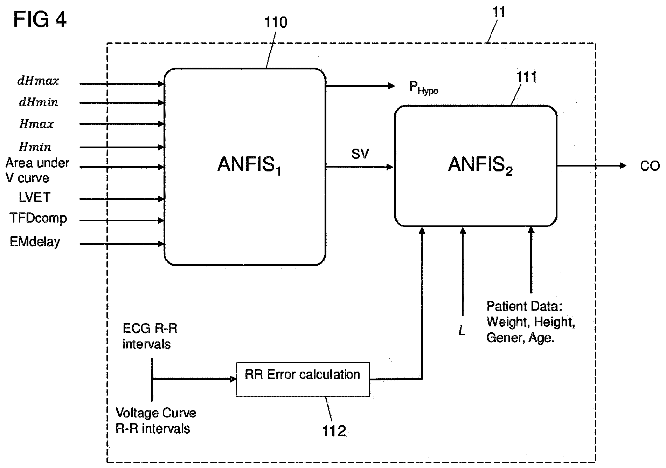

In particular, from the derivative DVC of the voltage curve VC, as shown in Fig. 2B, the left ventricular ejection time LVET can be estimated as the period from a point B, defined as the minimum of DVC prior to the maximum point C, to a point X, defined as the minimum of the DVC immediately after said point C. By integrating the voltage curve VC over the LVET period a value for the area F beneath the voltage curve VC over the interval of LEVT is obtained. The values LVET, dHmax, dHmin and the area F defined by said variables may be regarded as initial correlates of the stroke volume and are used as inputs to the model unit 11, as shown in Figs. 1 and 4.

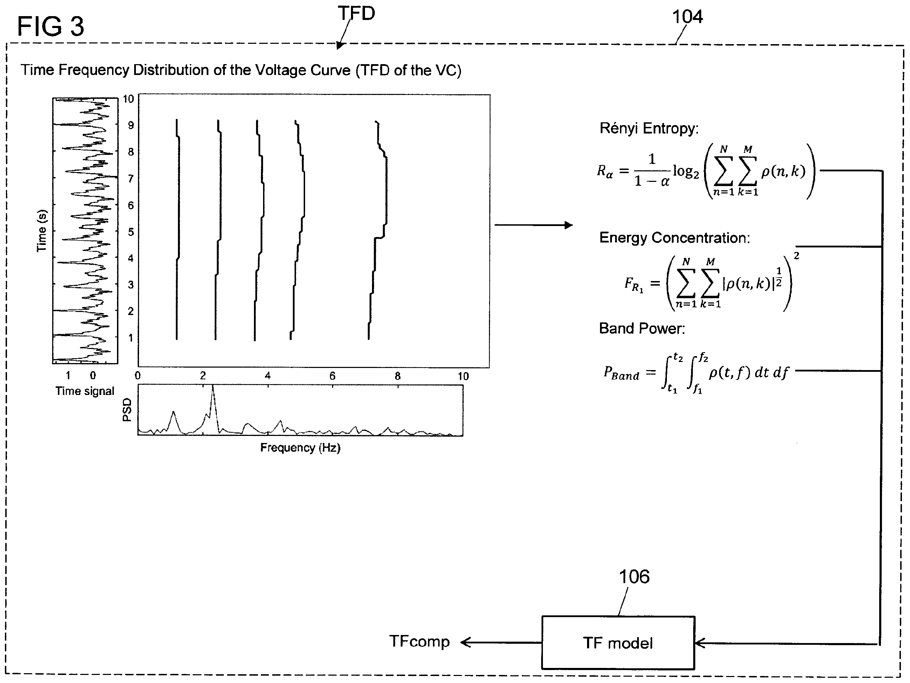

In addition, within the feature extraction unit 104, as illustrated in Fig. 3, time-frequency distributions TFD are determined from the bio-impedance measurement signal, the time-frequency distribution indicating the variation of the frequency spectrum of the bio-impedance measurement signal over time.

As illustrated in Fig. 3, from one or multiple time-frequency distributions of the bio-impedance measurement signal a number of different time-frequency features are obtained, such as the so-called Renyi entropy, the energy concentration and the band power defined by the equations indicated in Fig. 3, which each may be regarded as a correlate to the cardiac output of a patient. The different time-frequency features are, in the illustrated embodiment, fed to a generalized linear model 106 (for example a quadratic model) which is used to combine the different time-frequency features to obtain a combined value TFcomp, which then may be fed as a characteristic feature to the model unit 11.

In particular, using the derivative DVC of the impedance curve VC, p(t,f) may be calculated and may be used to compute the so-called Rényi entropy (the missing parameter $\alpha$ typically being chosen as an integer of 3 or more), the energy concentration and the band power (assuming appropriate band limits).

From the electrocardiogram signal E and the voltage signal VC, in particular the derivative DVC of the voltage signal VC, in addition a time difference between an R peak in the electrocardiogram signal E (see Fig. 2C) and an associated C peak in the derivative DVC of the voltage curve VC may be determined, corresponding to a time lag between the bio-impedance measurement signal and the electrocardiogram signal E. This is done in a comparison unit 105 as illustrated in Fig. 1 and is based on the background that the electrocardiogram signal E represents and is associated with the electrical function of the heart, whereas the bio-impedance measurement signal represents and is associated with mechanical changes in fluids and other tissues in the thorax. Thus, the time lag between the peaks R and C, corresponding to the so-called electromechanical (EM) delay, represents a correlate to the stroke volume and hence may be fed as a characteristic feature to the model unit 11.

The model unit 11 comprises two non-linear models 110, 111, as it is shown in Fig. 4. A first non-linear model 110 takes as input the parameters extracted from the bio-impedance measurement signal (voltage curve VC and its derivative DVC) and the electrocardiogram signal E. In a preferred embodiment, at least three inputs are used, selected for example from the group of input parameters to the first non-linear model 110 as illustrated in Fig 4. However, more than three input parameters and additionally other input parameters than the ones shown in Fig. 4 can potentially be used.

The first non-linear model 110 outputs an estimate for the stroke volume (SV) and in addition a hypotension index ($P_{Hypo}$) indicative of the probability of hypotension for the patient 2. The first non-linear model 110 feeds the estimate of the stroke volume to a second non-linear model 111. The second non-linear model 111 is being fed, as further input parameters, for example with a value L indicative of the length of the trunk of the patient 2 (corresponding for example to the distance between the lower and upper pair of electrodes 100E, 100S) and a value indicative of the RR interval. As additional inputs, the second non-linear model 111 may receive information relating to the patient 2, for example the gender, weight and/or age of the patient 2.

The RR interval (RR) corresponds to the reciprocal of the heart rate. The RR interval may be detected both from the voltage curve VC, in particular the derivative DVC of the voltage curve VC, and from the electrocardiogram signal E. Herein, to ensure correct performance and operation of the system, as illustrated in Fig. 4 the RR interval may be determined both from the voltage curve VC respectively the derivative DVC of the voltage curve VC and the electrocardiogram signal E, and in an error estimation unit 112 the different values determined for the RR interval may be compared to conduct a plausibility check. If it is found that the RR interval as determined as CC from the voltage curve VC and the RR interval as determined from the electrocardiogram signal E differ by more than a predetermined threshold, a correction mechanism may be activated in order to ensure that a correct value for the RR interval and the heart rate is used within the second non-linear model 111.

Generally, an estimate for the cardiac output (CO) can be calculated from the stroke volume (SV) as and the (momentary) RR interval

$$CO = SV / RR.$$

The acceptable range of the cardiac output CO may for example be 0 to 25 l/min (as compared to a "normal" physiological range of 4 to 8 l/min). The correlate of the cardiac output may be determined within the second non-linear model 111 or may be fed into the second non-linear model 111, in which the different input parameters are combined to output a final output value for the cardiac output (CO).

Within the embodiment described herein, the electrocardiogram signal is recorded and the RR interval and other characteristic features are extracted from the electrocardiogram signal. Furthermore, an FFT of the RR interval may be carried out, from which a value indicative of the heart rate variability (HRV) may be derived. From the heart rate variability, different frequency bands may be extracted, for example a high frequency range HF and a low frequency range LF, see Table 1 above. In addition or alternatively, other parameters may be extracted, such as a value RMSSD indicative of root mean square differences between successive RR intervals, a value SDSD indicative of the standard deviation of differences between successive RR intervals, and a value pNN50 indicative of a number of interval differences of successive RR intervals greater than 50 ms divided by the total number of RR intervals, as summarized in Table 1 above. Such additional features may be used as further inputs to the first non-linear model 110 and/or the second non-linear model 111.

The first non-linear model 110 may for example be a fuzzy logic model or a quadratic equation model, which combines the characteristic features and outputs an estimate of the stroke volume and potentially a value indicative of the so-called hypotension probability. Likewise, the second non-linear model 111 may for example be a fuzzy logic model or a quadratic equation model. The second model 111 aims at exploring the causal relationship between stroke volume and ECG activity, and integrates information from both in order to output a final index for the cardiac output CO.

Both models 110, 111 may take more or less inputs than described above.

The training of the non-linear models 110, 111 is beneficially carried out with a large amount of data where the stroke volume and cardiac output is known for the patient. The training defines the parameters of the models which can then predict the stroke volume and cardiac output when the inputs are presented to the model.

As said, for the processing non-linear models 110, 111 in the shape of fuzzy logic models or quadratic equation models may be employed. However, also other non-linear models may be used.

In the following, by way of example details about so-called Adaptive Neuro Fuzzy Inference System (ANFIS) models and quadratic equation models, which may be used for the first and/or second non-linear model 110, 111, are provided.

ANFIS model:

A fuzzy logic model may for example be the so-called Adaptive Neuro Fuzzy Inference System (ANFIS) model. In that case, the system 1 uses ANFIS models to combine the parameters, for the definition of the stroke volume and the cardiac output. The parameters extracted from the impedance and the ECG signals and the demographic data of the patient are used as input to an Adaptive Neuro Fuzzy Inference System (ANFIS).

ANFIS is a hybrid between a fuzzy logic system and a neural network. ANFIS does not assume any mathematical function governing the relationship between input and output. ANFIS applies a data driven approach where the training data decides the behaviour of the system.

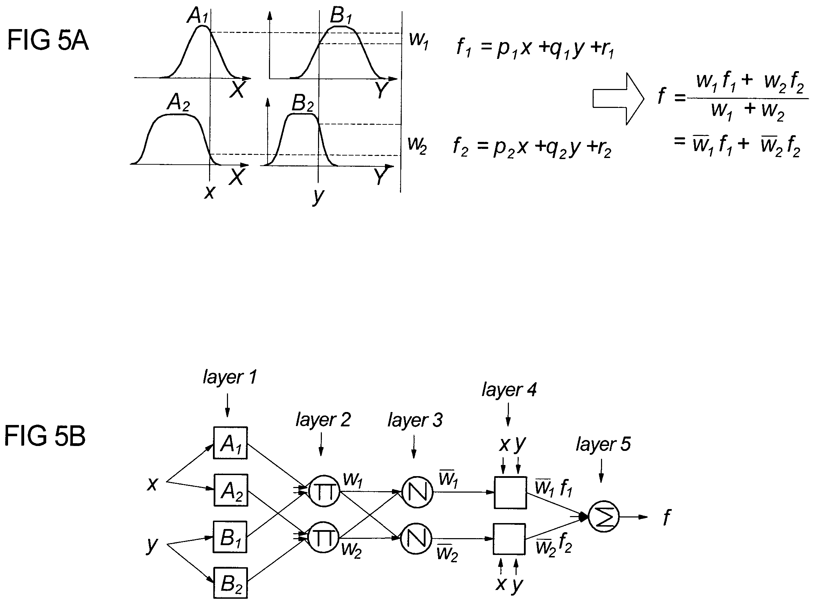

The five layers of ANFIS, shown in Fig. 5A and 5B, have the following functions:

- Each unit in Layer 1 stores three parameters to define a bell-shaped membership function. Each unit is connected to exactly one input unit and computes the membership degree of the input value obtained.
- Each rule is represented by one unit in Layer 2. Each unit is connected to those units in the previous layer, which are from the antecedent of the rule. The inputs into a unit are degrees of membership, which are multiplied to determine the degree of fulfilment for the rule represented.
- In Layer 3, for each rule there is a unit that computes its relative degree of fulfilment by means of a normalisation equation. Each unit is connected to all the rule units in Layer 2.
- The units of Layer 4 are connected to all input units and to exactly one unit in Layer 3. Each unit computes the output of a rule.
- An output unit in Layer 5 computes the final output by summing all the outputs from Layer 4.

Standard learning procedures from neural network theory are applied in ANFIS. Back-propagation is used to learn the antecedent parameters, i.e. the membership functions, and least squares estimation is used to determine the coefficients of the linear combinations in the rules' consequents. A step in the learning procedure has two passes. In the first pass, the forward pass, the input patterns are propagated, and the optimal consequent parameters are estimated by an iterative least mean squares procedure, while the antecedent parameters are fixed for the current cycle through the training set. In the second pass (the backward pass) the patterns are propagated again, and in this pass back-propagation is used to modify the antecedent parameters, while the consequent parameters remain fixed. This procedure is then iterated through the desired number of epochs. If the antecedent parameters initially are chosen appropriately, based on expert knowledge, one epoch is often sufficient as the LMS algorithm determines the optimal consequent parameters in one pass and if the antecedents do not change significantly by use of the gradient descent method, neither will the LMS calculation of the consequents lead to another result. For example in a 2-input, 2-rule system, rule 1 is defined by

$$if\ x\ is\ A\ and\ y\ is\ B\ then\ f_1 = p_1 x + q_1 y + r_1$$

where p, q and r are linear, termed consequent parameters or only consequents. Most common is f of first order as higher order Sugeno fuzzy models introduce great complexity with little obvious merit.

The inputs to the ANFIS system are fuzzified into a number of predetermined classes. The number of classes should be larger or equal two. The number of classes can be determined by different methods. In traditional fuzzy logic the classes are defined by an expert. The method can only be applied if it is evident to the expert where the landmarks between two classes can be placed. ANFIS optimizes the position of the landmarks, however the gradient descent method will reach its minimum faster if the initial value of the parameters defining the classes is close to the optimal values. By default, ANFIS initial landmarks are chosen by dividing the interval from minimum to maximum of all data into n equidistant intervals, where n is the number of classes. The number of classes could also be chosen by plotting the data in a histogram and visually deciding for an adequate number of classes, by ranking as done by FIR, through various clustering methods or Markov models. The ANFIS default was chosen for this invention and it showed that more than three classes resulted in instabilities during the validation phase, hence either two or three classes were used.

Both the number of classes and number of inputs add to the complexity of the model, i.e., the number of parameters. For example, in a system with four inputs each input may be fuzzified into three classes consisting of 36 antecedent (non-linear) and 405 consequent (linear) parameters, calculated by the following two formulas:

$$antecedents = number\ of\ classes\ x\ number\ of\ inputs\ x\ 3$$

$$consequents = number\ of\ classes\ number\ of\ inputs\ x\ (number\ of\ inputs\ +1)$$

The number of input-output pairs should in general be much larger (at least a factor 10) than the number of parameters in order to obtain a meaningful solution of the parameters.

A useful tool for ensuring stability is the experience obtained by working with a certain neuro-fuzzy system such as ANFIS in the context of a particular data set, and testing with extreme data for example obtained by simulation

ANFIS uses a Root Mean Square Error (RMSE) to validate the training result and from a set of validation data the RMSE validation error can be calculated after each training epoch. One epoch is defined as one update of both the antecedent and the consequent parameters. An increased number of epochs will in general decrease the training error.

QUADRATIC MODEL

Alternatively, quadrative equation models may be used for the models 110, 111. In that case, the system 1 uses quadratic models to combine the parameters for the definition of the stroke volume and the cardiac output. The parameters extracted from the impedance and the ECG signals and the demographic data of the patient are used as inputs to a quadratic model.

The output indexes are derived from quadratic generalized models that use as inputs data extracted from the ECG, impedance and demographic patient data. Such a model contains an independent coefficient called Intercept, one linear term per input, a square term per input and interaction terms between each pair of entries. The model can be expressed as:

$$Output = Intercept + \sum_{i=1}^{n} a_i * Input_i + \sum_{i=1}^{n} b_i * Input_i^2 + \sum_{j=1}^{n}\sum_{i=j+1}^{n} c_{j,i} * Input_i * Input_j$$

Where:

Intercept: intersection or constant term.
Input: input model.
Output: model output.
n: number of model inputs

**EP 3 684 249 B1**

a: linear terms.
b: square terms
c: interaction terms between inputs.

**List of Reference Numerals**

| 1 | System |
|---|---|
| 10 | Processing path |
| 100E | Excitation electrode |
| 100S | Sensing electrode |
| 101 | Amplification device |
| 102 | Analog-digital converter |
| 103, 104 | Feature extraction unit |
| 105 | Comparison unit |
| 106 | Model |
| 11 | Model unit |
| 110, 111 | Non-linear model |
| 112 | Error estimation unit |
| 12 | Processor device |
| 2 | Patient |
| 20 | Thorax |
| DVC | Derivative of voltage curve |
| E | ECG signal |
| R1, R2 | R-peak |
| TFD | Time-frequency distribution |
| VC | Measurement signal (Voltage curve) |

**Claims**

1. A system (1) for estimating the stroke volume and/or the cardiac output of a patient, comprising: a processor device (12) constituted to

- receive a bio-impedance measurement signal (VC) relating to a bio-impedance measurement on the thorax (2) of a patient (2),
- process the bio-impedance measurement signal (VC) to extract a group of characteristic features from the bio-impedance measurement signal (VC) and/or its derivative (DVC), and
- determine, using the group of extracted characteristic features, an output value indicative of the stroke volume and/or the cardiac output using at least one non-linear model (110, 111),

**characterized in**

**that** the system further comprises two excitation electrodes (100E) suitable for positioning on the thorax (20) of a patient (2) for applying an excitation signal, wherein the excitation electrodes (100E) are controlled to inject an electrical current having a constant amplitude of 50 to 1000 $\mu$A and a high frequency, for example 50 kHz, and two sensing electrodes (100S) suitable for positioning on the thorax (20) of the patient (2) for sensing the bio-impedance measurement signal (VC) caused by the excitation signal, one excitation electrode (100E) is placed at an upper position, close to the neck of the patient, and another excitation electrode (100E) is placed at a lower position on the thorax, with each sensing electrode (100S) being arranged in the vicinity of the associated excitation electrode (100E),
wherein the processor device (12) is constituted to process the bio-impedance measurement signal (VC) to compute at least one time-frequency distribution (TFD) based on the bio-impedance measurement signal (VC) and/or the derivative (DVC) of the bio-impedance measurement signal (VC) and to determine at least one characteristic feature of said group of characteristic features based on the at least one time-frequency distribution (TFD), and
wherein the processor device (12) is constituted to receive an electrocardiogram signal (ECG) and to process the electrocardiogram signal (ECG) to extract at least one characteristic feature, and

wherein the electrocardiogram signal (ECG) and the bio-impedance measurement signal (VC) are sensed using the same two common sensing electrodes (100S).

2. The system (1) according to claim 1, wherein the at least one time-frequency distribution (TFD) is computed according to the following equation:

$$\rho(t,f) = \iint G(t-u,\tau)z\left(u+\frac{\tau}{2}\right)\overline{z}\left(u-\frac{\tau}{2}\right)dud\tau$$

in which $\rho$ represents the time-frequency distribution, $G(t, \tau)$ represents a time-lag kernel, z represents the analytic associate of the bio-impedance measurement signal (VC) to be analysed, and $\overline{z}$ represents the complex conjugate of z.

3. The system (1) according to claim 1 or 2, wherein, for determining said at least one characteristic feature of said group of characteristic features, the processor device (12) is constituted to determine, based on the at least one time-frequency distribution (TFD), at least one time-frequency distribution feature, including at least one of the group of a value indicative of the time-frequency complexity, a value indicative of the time-frequency Renyi entropy, a value indicative of the normalized time-frequency Renyi entropy, a value indicative of the energy distribution measure, and a value indicative of the energy of at least one band.

4. The system (1) according to claim 3, wherein, for determining said at least one characteristic feature of said group of characteristic features, the processor device (12) is constituted to determine at least two time-frequency distribution features and to combine said at least two time-frequency distribution features to obtain a characteristic feature.

5. The system according to one of the proceeding claims, wherein the processor device (12) is constituted to process said bio-impedance measurement signal (VC) and said electrocardiogram signal (ECG) in a processing path (10) comprising an amplification device (101) for amplifying the electrocardiogram signal (ECG) and the bio-impedance measurement signal (VC) and an analog-to-digital converter (112) for digitizing the electrocardiogram signal (ECG) and the bio-impedance measurement signal (VC).

6. The system according to one of the preceding claims, wherein the processor device (12) is constituted to extract at least one of the group of a maximum value (dHmax) of the derivative (DVC) of the bio-impedance measurement signal (VC), a minimum value (dHmin) of the derivative (DVC) of the bio-impedance measurement signal (VC), a maximum amplitude (Hmax) of the bio-impedance measurement signal (VC), a minimum amplitude (Hmin) of the bio-impedance measurement signal (VC), a value of the left ventricular ejection time (LVET) derived from the derivative of the bio-impedance measurement signal (VC), an area (F) obtained by integrating the derivative (DVC) of the voltage curve (VC) over the left ventricular ejection time (LVET), and a value (EMdelay) indicative of a time difference of a C peak in the derivative of the bio-impedance measurement signal (VC) and an R peak of an electrocardiogram signal (ECG), to obtain the group of extracted characteristic features.

7. The system (1) according to one of the preceding claims, wherein the processor device (12) is constituted to feed the group of extracted characteristic features into a first non-linear model (110), in particular a first fuzzy logic model or a first quadratic equation model, the first non-linear model (110) being constituted to output a value indicative of the stroke volume.

8. The system according to claim 7, wherein the processor device (12) is constituted to determine a correlate of the cardiac output by multiplying the value indicative of the stroke volume with a value indicative of the heart rate of the patient.

9. The system according to claim 8, wherein the processor device (12) is constituted to derive said value indicative of the heart rate from an electrocardiogram signal (ECG) and/or said bio-impedance measurement signal (VC).

10. The system according to one of claims 7 to 9, wherein the processor device (12) is constituted to feed the value indicative of the stroke volume into a second non-linear model (111), in particular a second fuzzy logic model or a second quadratic equation model, the second non-linear model (111) being constituted to output a final output value indicative of the stroke volume and/or a final output value indicative of the cardiac output.

11. The system according to claim 10, wherein the processor device (12) is constituted to feed, as further input, information relating to the patient's (2) weight, height, gender, and/or age into the second non-linear model (111).

12. A method for estimating the stroke volume and/or the cardiac output of a patient, comprising:

> - receiving a bio-impedance measurement signal (VC) relating to a bio-impedance measurement on the thorax (2) of a patient (2),
> - processing the bio-impedance measurement signal (VC) to extract a group of characteristic features from the bio-impedance measurement signal (VC) and/or its derivative (DVC), and
> - determining, using the group of extracted characteristic features, an output indicative of the stroke volume and/or the cardiac output using at least one non-linear model (110, 111),

> **characterized in**

> **that** further comprising the step of positioning two excitation electrodes (100E) on the thorax (20) of a patient (2) for applying an excitation signal, wherein the excitation electrodes (100E) are controlled to inject an electrical current having a constant amplitude of 50 to 1000 µA and a high frequency, for example 50 kHz, and at least two sensing electrodes (100S) to be placed on the thorax (20) of the patient (2) for sensing the bio-impedance measurement signal (VC) caused by the excitation signal, one excitation electrode (100E) is placed at an upper position, close to the neck of the patient, and another excitation electrode (100E) is placed at a lower position on the thorax, with each sensing electrode (100S) being arranged in the vicinity of the associated excitation electrode (100E), and the step of
> receiving by the processor device (12) electrocardiogram signal (ECG) and processing the electrocardiogram signal (ECG) to extract at least one characteristic feature, wherein the electrocardiogram signal (ECG) and the bio-impedance measurement signal (VC) are sensed using the same two common sensing electrodes (100S), and
> wherein

> the processing of the bio-impedance measurement signal (VC) includes: processing the bio-impedance measurement signal (VC) to compute at least one time-frequency distribution (TFD) based on the bio-impedance measurement signal (VC) and/or the derivative (DVC) of the bio-impedance measurement signal (VC), and determining at least one characteristic feature of said group of characteristic features based on the at least one time-frequency distribution (TFD).

**Patentansprüche**

1. System (1) zur Schätzung des Schlagvolumens und/oder der Herzleistung eines Patienten, umfassend: eine Prozessorvorrichtung (12), die zu Folgendem ausgebildet ist:

> - Empfangen eines Bioimpedanzmesssignals (VC), das sich auf eine Bioimpedanzmessung am Thorax (2) eines Patienten (2) bezieht,
> - Verarbeiten des Bioimpedanzmesssignals (VC), um eine Gruppe von charakteristischen Merkmalen aus dem Bioimpedanzmesssignal (VC) und/oder seiner Ableitung (DVC) zu extrahieren, und
> - Bestimmen, unter Verwendung der Gruppe von extrahierten charakteristischen Merkmalen, eines Ausgabewertes, der das Schlagvolumen und/oder die Herzleistung angibt, unter Verwendung mindestens eines nichtlinearen Modells (110, 111),

> **dadurch gekennzeichnet,**

> **dass** das System ferner zwei Anregungselektroden (100E), die geeignet sind, auf dem Thorax (20) eines Patienten (2) positioniert zu werden, um ein Anregungssignal anzulegen, wobei die Anregungselektroden (100E) gesteuert werden, um einen elektrischen Strom mit einer konstanten Amplitude von 50 bis 1000 µA und einer hohen Frequenz, beispielsweise 50 kHz, zu injizieren, und zwei Sensorelektroden (100S) umfasst, die geeignet sind, auf dem Thorax (20) des Patienten (2) positioniert zu werden, um das durch das Anregungssignal verursachte Bioimpedanzmesssignal (VC) zu erfassen, wobei eine Anregungselektrode (100E) an einer oberen Position nahe dem Hals des Patienten platziert ist und eine andere Anregungselektrode (100E) an einer unteren Position auf dem Thorax platziert ist, wobei jede Sensorelektrode (100S) in der Nähe der zugeordneten Anregungselektrode (100E) angeordnet ist,

wobei die Prozessorvorrichtung (12) dazu ausgebildet ist, das Bioimpedanzmesssignal (VC) zu verarbeiten, um mindestens eine Zeit-Frequenz-Verteilung (TFD) basierend auf dem Bioimpedanzmesssignal (VC) und/oder der Ableitung (DVC) des Bioimpedanzmesssignals (VC) zu berechnen und mindestens ein charakteristisches Merkmal der Gruppe von charakteristischen Merkmalen basierend auf der mindestens einen Zeit-Frequenz-Verteilung (TFD) zu bestimmen, und

wobei die Prozessorvorrichtung (12) dazu ausgebildet ist, ein Elektrokardiogrammsignal (ECG) zu empfangen und das Elektrokardiogrammsignal (ECG) zu verarbeiten, um mindestens ein charakteristisches Merkmal zu extrahieren, und

wobei das Elektrokardiogrammsignal (ECG) und das Bioimpedanzmesssignal (VC) unter Verwendung der gleichen zwei gemeinsamen Sensorelektroden (100S) erfasst werden.

2.  System (1) nach Anspruch 1, wobei die mindestens eine Zeit-Frequenz-Verteilung (TFD) nach der folgenden Gleichung berechnet wird:

$$\rho(t, f) = \iint G(t - u, \tau) z\left(u + \frac{\tau}{2}\right) \overline{z}\left(u - \frac{\tau}{2}\right) du d\tau$$

in der $p$ die Zeit-Frequenz-Verteilung darstellt, $G(t, \tau)$ einen Zeitverzögerungs-Kernel darstellt, z den analytischen Begleiter des zu analysierenden Bioimpedanzmesssignals (VC) darstellt und $\overline{z}$ die komplexe Konjugierte von z darstellt.

3.  System (1) nach Anspruch 1 oder 2, wobei zum Bestimmen des mindestens einen charakteristischen Merkmals der Gruppe von charakteristischen Merkmalen die Prozessorvorrichtung (12) dazu ausgebildet ist, basierend auf der mindestens einen Zeit-Frequenz-Verteilung (TFD), mindestens ein Zeit-Frequenz-Verteilungsmerkmal zu bestimmen, einschließlich mindestens eines aus der Gruppe eines Wertes, der die Zeit-Frequenz-Komplexität angibt, eines Wertes, der die Zeit-Frequenz-Renyi-Entropie angibt, eines Wertes, der die normalisierte Zeit-Frequenz-Renyi-Entropie angibt, eines Wertes, der das Energieverteilungsmaß angibt, und eines Wertes, der die Energie von mindestens einem Band angibt.

4.  System (1) nach Anspruch 3, wobei zum Bestimmen des mindestens einen charakteristischen Merkmals der Gruppe von charakteristischen Merkmalen die Prozessorvorrichtung (12) dazu ausgebildet ist, mindestens zwei Zeit-Frequenz-Verteilungsmerkmale zu bestimmen und die mindestens zwei Zeit-Frequenz-Verteilungsmerkmale zu kombinieren, um ein charakteristisches Merkmal zu erhalten.

5.  System nach einem der vorhergehenden Ansprüche, wobei die Prozessorvorrichtung (12) dazu ausgebildet ist, das Bioimpedanzmesssignal (VC) und das Elektrokardiogrammsignal (ECG) in einem Verarbeitungspfad (10) zu verarbeiten, der eine Verstärkungsvorrichtung (101) zum Verstärken des Elektrokardiogrammsignals (ECG) und des Bioimpedanzmesssignals (VC) und einen Analog-Digital-Wandler (112) zum Digitalisieren des Elektrokardiogrammsignals (ECG) und des Bioimpedanzmesssignals (VC) umfasst.

6.  System nach einem der vorhergehenden Ansprüche, wobei die Prozessorvorrichtung (12) dazu ausgebildet ist, mindestens einen aus der Gruppe eines maximalen Wertes (dHmax) der Ableitung (DVC) des Bioimpedanzmesssignals (VC), eines minimalen Wertes (dHmin) der Ableitung (DVC) des Bioimpedanzmesssignals (VC), einer maximalen Amplitude (Hmax) des Bioimpedanzmesssignals (VC), einer minimalen Amplitude (Hmin) des Bioimpedanzmesssignals (VC), einen Wert der linksventrikulären Ejektionszeit (LVET), der aus der Ableitung des Bioimpedanzmesssignals (VC) abgeleitet wird, eine Fläche (F), die durch Integration der Ableitung (DVC) der Spannungskurve (VC) über die linksventrikuläre Ejektionszeit (LVET) erhalten wird, und einen Wert (EMVerzögerung), der eine Zeitdifferenz zwischen einem C-Peak in der Ableitung des Bioimpedanzmesssignals (VC) und einem R-Peak eines Elektrokardiogrammsignals (ECG) angibt, zu extrahieren, um die Gruppe der extrahierten charakteristischen Merkmale zu erhalten.

7.  System (1) nach einem der vorhergehenden Ansprüche, wobei die Prozessorvorrichtung (12) dazu ausgebildet ist, die Gruppe von extrahierten charakteristischen Merkmalen in ein erstes nichtlineares Modell (110), insbesondere in ein erstes Fuzzy-Logik-Modell oder ein erstes quadratisches Gleichungsmodell, einzuspeisen, wobei das erste nichtlineare Modell (110) dazu ausgebildet ist, einen Wert auszugeben, der das Schlagvolumen angibt.

8. System nach Anspruch 7, wobei die Prozessorvorrichtung (12) dazu ausgebildet ist, ein Korrelat der Herzleistung zu bestimmen, indem der Wert, der das Schlagvolumen angibt, mit einem Wert multipliziert wird, der die Herzfrequenz des Patienten angibt.

9. System nach Anspruch 8, wobei die Prozessorvorrichtung (12) dazu ausgebildet ist, den die Herzfrequenz angebenden Wert aus einem Elektrokardiogrammsignal (ECG) und/oder dem Bioimpedanzmesssignal (VC) abzuleiten.

10. System nach einem der Ansprüche 7 bis 9, wobei die Prozessorvorrichtung (12) dazu ausgebildet ist, den das Schlagvolumen angebenden Wert in ein zweites nichtlineares Modell (111), insbesondere ein zweites Fuzzy-Logik-Modell oder ein zweites quadratisches Gleichungsmodell, einzuspeisen, wobei das zweite nichtlineare Modell (111) dazu ausgebildet ist, einen das Schlagvolumen angebenden Endausgabewert und/oder einen die Herzleistung angebenden Endausgabewert auszugeben.

11. System nach Anspruch 10, wobei die Prozessorvorrichtung (12) dazu ausgebildet ist, als weitere Eingabe Informationen in Bezug auf das Gewicht, die Größe, das Geschlecht und/oder das Alter des Patienten (2) in das zweite nichtlineare Modell (111) einzuspeisen.

12. Verfahren zur Schätzung des Schlagvolumens und/oder der Herzleistung eines Patienten, umfassend:

   - Empfangen eines Bioimpedanzmesssignals (VC), das sich auf eine Bioimpedanzmessung am Thorax (2) eines Patienten (2) bezieht,
   - Verarbeiten des Bioimpedanzmesssignals (VC), um eine Gruppe von charakteristischen Merkmalen aus dem Bioimpedanzmesssignal (VC) und/oder seiner Ableitung (DVC) zu extrahieren, und
   - Bestimmen, unter Verwendung der Gruppe von extrahierten charakteristischen Merkmalen, einer Ausgabe, die das Schlagvolumen und/oder die Herzleistung angibt, unter Verwendung mindestens eines nichtlinearen Modells (110, 111),

   **dadurch gekennzeichnet,**

   **dass** es ferner den Schritt des Positionierens von zwei Anregungselektroden (100E) auf dem Thorax (20) eines Patienten (2), um ein Anregungssignal anzulegen, wobei die Anregungselektroden (100E) gesteuert werden, um einen elektrischen Strom mit einer konstanten Amplitude von 50 bis 1000 µA und einer hohen Frequenz, beispielsweise 50 kHz, zu injizieren, und von mindestens zwei Sensorelektroden (100S), die auf dem Thorax (20) des Patienten (2) zu platzieren sind, um das durch das Anregungssignal verursachte Bioimpedanzmesssignal (VC) zu erfassen, wobei eine Anregungselektrode (100E) an einer oberen Position nahe dem Hals des Patienten platziert ist und eine andere Anregungselektrode (100E) an einer unteren Position auf dem Thorax platziert ist, wobei jede Sensorelektrode (100S) in der Nähe der zugeordneten Anregungselektrode (100E) angeordnet ist, und den Schritt des Empfangens eines Elektrokardiogrammsignals (ECG) durch die Prozessorvorrichtung (12) und des Verarbeitens des Elektrokardiogrammsignals (ECG) umfasst, um mindestens ein charakteristisches Merkmal zu extrahieren, wobei das Elektrokardiogrammsignal (ECG) und das Bioimpedanzmesssignal (VC) unter Verwendung der gleichen zwei gemeinsamen Sensorelektroden (100S) erfasst werden, und wobei das Verarbeiten des Bioimpedanzmesssignals (VC) Folgendes einschließt: Verarbeiten des Bioimpedanzmesssignals (VC), um mindestens eine Zeit-Frequenz-Verteilung (TFD) basierend auf dem Bioimpedanzmesssignal (VC) und/oder der Ableitung (DVC) des Bioimpedanzmesssignals (VC) zu berechnen, und Bestimmen mindestens eines charakteristischen Merkmals der Gruppe von charakteristischen Merkmalen basierend auf der mindestens einen Zeit-Frequenz-Verteilung (TFD).

## Revendications

1. Système (1) permettant d'estimer le volume systolique et/ou le débit cardiaque d'un patient, comprenant : un dispositif de traitement (12) constitué pour

   - recevoir un signal de mesure de bio-impédance (VC) relatif à une mesure de bio-impédance sur le thorax (2) d'un patient (2),
   - traiter le signal de mesure de bio-impédance (VC) pour extraire un groupe d'éléments caractéristiques du signal de mesure de bio-impédance (VC) et/ou de sa dérivée (DVC), et

- déterminer, à l'aide du groupe d'éléments caractéristiques extraites, une valeur de sortie indicative du volume systolique et/ou du débit cardiaque à l'aide d'au moins un modèle non linéaire (110, 111),

**caractérisé en ce**

**que** le système comprend en outre deux électrodes d'excitation (100E) appropriées pour être positionnées sur le thorax (20) d'un patient (2) pour l'application d'un signal d'excitation, dans lequel les électrodes d'excitation (100E) sont commandées pour injecter un courant électrique ayant une amplitude constante de 50 à 1000 μA et une fréquence élevée, par exemple 50 kHz, et deux électrodes de détection (100S) appropriées pour être positionnées sur le thorax (20) du patient (2) pour la détection du signal de mesure de bio-impédance (VC) provoqué par le signal d'excitation, une électrode d'excitation (100E) est placée au niveau d'une position supérieure, près du cou du patient, et une autre électrode d'excitation (100E) est placée au niveau d'une position plus basse sur le thorax, avec chaque électrode de détection (100S) étant agencée à proximité de l'électrode d'excitation (100E) associée,

dans lequel le dispositif de traitement (12) est constitué pour traiter le signal de mesure de bio-impédance (VC) pour calculer au moins une distribution temps-fréquence (TFD) en fonction du signal de mesure de bio-impédance (VC) et/ou la dérivée (DVC) du signal de mesure de bio-impédance (VC) et pour déterminer au moins un élément caractéristique dudit groupe d'éléments caractéristiques en fonction de l'au moins une distribution temps-fréquence (TFD), et

dans lequel le dispositif de traitement (12) est constitué pour recevoir un signal d'électrocardiogramme (ECG) et pour traiter le signal d'électrocardiogramme (ECG) pour extraire au moins un élément caractéristique, et

dans lequel le signal d'électrocardiogramme (ECG) et le signal de mesure de bio-impédance (VC) sont détectés à l'aide des deux mêmes électrodes de détection (100S) communes.

2. Système (1) selon la revendication 1, dans lequel l'au moins une distribution temps-fréquence (TFD) est calculée selon l'équation suivante :

$$\rho(t, f) = \iint G(t - u, \tau) z\left(u + \frac{\tau}{2}\right) \bar{z}\left(u - \frac{\tau}{2}\right) du d\tau$$

où $p$ représente la distribution temps-fréquence, $G(t, \tau)$ représente un noyau à retardement, z représente l'associé analytique du signal de mesure de bio-impédance (VC) à analyser, et $\bar{z}$ représente le conjugué complexe de z.

3. Système (1) selon la revendication 1 ou 2, dans lequel, pour la détermination dudit au moins un élément caractéristique dudit groupe d'éléments caractéristiques, le dispositif de traitement (12) est constitué pour déterminer, en fonction de l'au moins une distribution temps-fréquence (TFD), au moins un élément de distribution temps-fréquence, comportant au moins l'un du groupe d'une valeur indicative de la complexité temps-fréquence, d'une valeur indicative de l'entropie de Renyi temps-fréquence, d'une valeur indicative de l'entropie de Renyi normalisée temps-fréquence, d'une valeur indicative de la mesure de distribution d'énergie, et une valeur indicative de l'énergie d'au moins une bande.

4. Système (1) selon la revendication 3, dans lequel, pour la détermination dudit au moins un élément caractéristique dudit groupe d'éléments caractéristiques, le dispositif de traitement (12) est constitué pour déterminer au moins deux caractéristiques de distribution temps-fréquence et pour combiner lesdits au moins deux éléments de distribution temps-fréquence pour obtenir un élément caractéristique.

5. Système selon l'une des revendications précédentes, dans lequel le dispositif de traitement (12) est constitué pour traiter ledit signal de mesure de bio-impédance (VC) et ledit signal d'électrocardiogramme (ECG) dans une voie de traitement (10) comprenant un dispositif d'amplification (101) pour l'amplification du signal d'électrocardiogramme (ECG) et le signal de mesure de bio-impédance (VC) et un convertisseur analogique-numérique (112) pour la numérisation du signal d'électrocardiogramme (ECG) et du signal de mesure de bio-impédance (VC).

6. Système selon l'une des revendications précédentes, dans lequel le dispositif de traitement (12) est constitué pour extraire au moins l'un du groupe d'une valeur maximale (dHmax) de la dérivée (DVC) du signal de mesure de bio-impédance (VC), d'une valeur minimale (dHmin) de la dérivée (DVC) du signal de mesure de bio-impédance (VC), d'une amplitude maximale (Hmax) du signal de mesure de bio-impédance (VC), d'une amplitude minimale (Hmin) du signal de mesure de bio-impédance (VC), d'une valeur du temps d'éjection ventriculaire gauche (TEVG) dérivée de la dérivée du signal de mesure de bio-impédance (VC), d'une aire (F) obtenue en intégrant la dérivée (DVC) de la courbe de tension (VC) sur le temps d'éjection ventriculaire gauche (LVET), et d'une valeur (EMdelay) indicative d'une différence de temps entre un pic C dans la dérivée du signal de mesure de bio-impédance (VC) et un pic R d'un signal d'électrocardiogramme (ECG), pour obtenir le groupe d'éléments caractéristiques extraites.

7. Système (1) selon l'une des revendications précédentes, dans lequel le dispositif de traitement (12) est constitué pour introduire le groupe d'éléments caractéristiques extraits dans un premier modèle non linéaire (110), en particulier un premier modèle de logique floue ou un premier modèle d'équation quadratique, le premier modèle non linéaire (110) étant constitué pour délivrer en sortie une valeur indicative du volume systolique.

8. Système selon la revendication 7, dans lequel le dispositif de traitement (12) est constitué pour déterminer un corrélat du débit cardiaque en multipliant la valeur indicative du volume systolique par une valeur indicative de la fréquence cardiaque du patient.

9. Système selon la revendication 8, dans lequel le dispositif de traitement (12) est constitué pour dériver ladite valeur indicative de la fréquence cardiaque à partir d'un signal d'électrocardiogramme (ECG) et/ou d'un signal de mesure de bio-impédance (VC).

10. Système selon l'une quelconque des revendications 7 à 9, dans lequel le dispositif de traitement (12) est constitué pour introduire la valeur indicative du volume systolique dans un second modèle non linéaire (111), en particulier un second modèle de logique floue ou un second modèle d'équation quadratique, le second modèle non linéaire (111) étant constitué pour délivrer en sortie une valeur de sortie finale indicative du volume systolique et/ou une valeur de sortie finale indicative du débit cardiaque.

11. Système selon la revendication 10, dans lequel le dispositif de traitement (12) est constitué pour introduire, en tant que données supplémentaires, des informations relatives au poids, à la taille, au sexe et/ou à l'âge du patient (2) dans le second modèle non linéaire (111).

12. Procédé permettant d'estimer le volume systolique et/ou le débit cardiaque d'un patient, comprenant :

- la réception d'un signal de mesure de bio-impédance (VC) relatif à une mesure de bio-impédance sur le thorax (2) d'un patient (2),
- le traitement du signal de mesure de bio-impédance (VC) pour extraire un groupe d'éléments caractéristiques du signal de mesure de bio-impédance (VC) et/ou de sa dérivée (DVC), et
- la détermination, à l'aide du groupe d'éléments caractéristiques extraites, d'une sortie indicative du volume systolique et/ou du débit cardiaque à l'aide d'au moins un modèle non linéaire (110, 111),

**caractérisé en ce**

**qu'**il comprend en outre l'étape consistant à positionner deux électrodes d'excitation (100E) sur le thorax (20) d'un patient (2) pour l'application d'un signal d'excitation, dans lequel les électrodes d'excitation (100E) sont commandées pour injecter un courant électrique ayant une amplitude constante de 50 à 1000 μA et une fréquence élevée, par exemple 50 kHz, et au moins deux électrodes de détection (100S) à placer sur le thorax (20) du patient (2) pour la détection du signal de mesure de bio-impédance (VC) provoqué par le signal d'excitation, une électrode d'excitation (100E) est placée au niveau d'une position supérieure, près du cou du patient, et une autre électrode d'excitation (100E) est placée au niveau d'une position plus basse sur le thorax, avec chaque électrode de détection (100S) étant agencée à proximité de l'électrode d'excitation (100E) associée, et l'étape consistant à

recevoir par le dispositif de traitement (12) un signal d'électrocardiogramme (ECG) et traiter le signal d'électrocardiogramme (ECG) pour extraire au moins un élément caractéristique, dans lequel le signal d'électrocardiogramme (ECG) et le signal de mesure de bio-impédance (VC) sont détectés à l'aide des deux mêmes électrodes de détection (100S) communes, et

dans lequel

le traitement du signal de mesure de bio-impédance (VC) comporte : le traitement du signal de mesure de bio-impédance (VC) pour calculer au moins une distribution temps-fréquence (TFD) en fonction du signal de mesure de bio-impédance (VC) et/ou la dérivée (DVC) du signal de mesure de bio-impédance (VC), et la détermination d'au moins un élément caractéristique dudit groupe d'éléments caractéristiques en fonction de l'au moins une distribution temps-fréquence (TFD).

FIG 1

FIG 2A — Voltage (V) — VC, Hmax, Hmin, CC

FIG 2B — -dV/dt — F, C, dHmax, dHmin, A, B, X, Y, O, LVET, EMdelay, DVC, C

FIG 2C — ECG — R, R, RR, ECG

# FIG 3

Time Frequency Distribution of the Voltage Curve (TFD of the VC)

TFD

104

Rényi Entropy:

$$R_\alpha = \frac{1}{1-\alpha} \log_2 \left( \sum_{n=1}^{N} \sum_{k=1}^{M} \rho(n,k) \right)$$

Energy Concentration:

$$F_{R_1} = \left( \sum_{n=1}^{N} \sum_{k=1}^{M} |\rho(n,k)|^{\frac{1}{2}} \right)^2$$

Band Power:

$$P_{Band} = \int_{t_1}^{t_2} \int_{f_1}^{f_2} \rho(t,f) \, dt \, df$$

106

TFcomp ← TF model

EP 3 684 249 B1

FIG 4

FIG 5A

$$f_1 = p_1 x + q_1 y + r_1$$

$$f = \frac{w_1 f_1 + w_2 f_2}{w_1 + w_2}$$

$$f_2 = p_2 x + q_2 y + r_2$$

$$= \overline{w}_1 f_1 + \overline{w}_2 f_2$$

FIG 5B

EP 3 684 249 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3340867 A
- US 3835840 A

- WO 2015086020 A1

**Non-patent literature cited in the description**

- **B. BOASHASH**. Time-Frequency Signal Analysis and Processing: a Comprehensive Reference. Elsevier, 2003